# EUROPÄISCHE PATENTSCHRIFT

(11) **EP 2 602 606 B1**
(45) Veröffentlichungstag und Bekanntmachung des Hinweises auf die Patenterteilung: **03.02.2016**
(21) Anmeldenummer: 12194016.7
(22) Anmeldetag: 23.11.2012
(51) Int. Cl.: G01N 3/56, G01N 33/42

(54) **Verfahren und Vorrichtung zum Feststellen des Abriebes eines Straßenbelages durch Spikereifen**
Method and appartus for determining the wear of a road surfacing due to studded tyres
Procédé et dispositif de détermination de la force d'un revêtement de route par des pneus à clous

(30) Priorität: 08.12.2011 DE 102011056158
(43) Veröffentlichungstag der Anmeldung: 12.06.2013
(73) Patentinhaber: Continental Reifen Deutschland GmbH, 30165 Hannover (DE)
(72) Erfinder: Brandau, Christian, 30419 Hannover (DE); Schlittenhard, Jan, 30900 Wedemark (DE); Bolz, Gerrit, 31535 Hannover (DE); Gültinger, Johannes, 76131 Karlsruhe (DE)
(74) Vertreter: Finger, Karsten

(56) Entgegenhaltungen:
- WO-A1-88/09491
- DE-A1- 2 611 123
- DE-B1- 2 148 362
- US-A- 4 938 055
- "Innentrommelprüfstand (IPS)", Bundesanstalt für Straßenwesen , 1. März 2009 (2009-03-01), XP055056601, Gefunden im Internet: URL:http://www.bast.de/cln_033/nn_37280/Sh aredDocs/Publikationen/infos-versuchseinri chtungen/IPS,templateId=raw,property=publi cationFile.pdf/IPS.pdf [gefunden am 2013-03-14]

## Beschreibung

Die Erfindung betrifft ein Verfahren zum Feststellen des Abriebes eines Straßenbelages durch Spikereifen in einem Prüflauf durch Überfahren des zu prüfenden Belages, welcher sich auf der Innenfläche einer in Drehbewegung versetzten Trommel befindet. Die Erfindung betrifft ferner eine Vorrichtung zur Durchführung des Verfahrens.

Es ist bekannt, zur Ermittlung des Abriebes eines Straßenbelages durch Spikereifen in eine Straßenoberfläche lokal Prüfbeläge einzusetzen, die anschließend mehrmals von einem Auto, welches mit Spikereifen versehen ist, überfahren werden. Die Prüfbeläge werden vor und nach dem Test gewogen, der Massenverlust entspricht dem Abrieb. Diese Testmethode ist sehr zeitaufwändig und daher sehr teuer und ist nicht ganzjährig durchführbar, da Außentemperaturen zwischen 0°C und 15°C erforderlich sind. Bei Temperaturen unter 0°C ist die erforderliche Bewässerung des Straßenbelages nicht mehr möglich, bei Temperaturen über 15°C wird der Asphalt zu weich, sodass sich um die Spikepins abgeriebener Asphalt ansammelt, wodurch die Messung verfälscht wird. Es ist ferner eine Testmethode bekannt, bei der zur Ermittlung des Abriebs von Straßenbelag durch Spikereifen die Innenfläche einer Trommel eines Trommelprüfstandes mit dem zu prüfenden Beleg komplett ausgekleidet wird. Es wird anhand der Konturvermessung der derart gebildeten umlaufenden Fahrbahn auf den Abrieb geschlossen. Für diese Methode ist eine Vielzahl von Überrollungen erforderlich, was sie ebenfalls zeitaufwändig und teuer macht.

Aus der Publikation "Innentrommelprüfstand (IPS)" der Bundesanstalt für Straßenwesen vom 1. März 2009 ist ein Prüfstand bekannt, welcher im Wesentlichen aus einer mechanisch gelagerten und einseitig offenen Lauftrommel besteht. In zwölf auswechselbaren Kassetten können reale Straßenbelege mit einer Stärke bis zu 80 mm in die Trommel eingebaut werden. Aus der US 4,938,055 A ist eine Vorrichtung zum Feststellen des Abriebes eines Straßenbelages bekannt, welche eine waagrecht montierte Scheibe aufweist, die in Drehbewegung versetzbar ist. Eine Anzahl von zu einem Kreisring zusammengesetzten Belagsplatten wird in Vertiefungen der rotierbaren Scheibe eingesetzt. Bei beiden bekannten Vorrichtungen ergeben daher die eingesetzten Prüfbelagsteile einen einheitlichen Prüfbelag, auf welchem der Reifen abgerollt wird.

Der Erfindung liegt die Aufgabe zugrunde, ein Prüfverfahren der eingangs genannten Art zur Verfügung zu stellen, welches mit geringem Zeitaufwand und kostengünstig die Ermittlung des Abriebes eines Straßenbelages durch Spikereifen gestattet.

Die gestellte Aufgabe wird erfindungsgemäß dadurch gelöst, dass auf die Innenfläche der Trommel ein herkömmlichen Asphaltbelag aufgebracht wird, welcher an zumindest einer Stelle im Umfang der Innenfläche unterbrochen wird, wobei der zu prüfende, vom Fahrbahnbelag abweichend ausgeführte Belag Gestein oder in einer Matrix eingebetteten Gesteinsbruch oder Kies enthält und als Belagsplatte oder Belagsplatten lediglich lokal an dieser Stelle bzw. diesen Stellen der Innenfläche der Trommel wieder entfernbar eingesetzt wird und vor und nach einem Prüflauf gewogen wird.

Die Erfindung beruht auf der Erkenntnis, dass der zu prüfende Belag lediglich an einer lokal begrenzten Stelle der Innenfläche der Trommel positioniert sein muss, um einem Abriebstest unterzogen zu werden. Innerhalb kürzester Zeit wird diese Stelle während der Drehung der Trommel vom Spikereifen sehr oft überrollt. Die Prüfläufe dauern nur wenige Minuten. Auch bei dieser Methode entspricht der Massenverlust des bzw. der Belagsplatte(n) dem zu erwartenden Abrieb. Das Wiegen des/der Belagsplatte(n) gestaltet sich einfach und schnell. Da solche Prüfstände in Räumen untergebracht sind, sind die Prüfläufe von äußeren Klimabedingungen unabhängig durchführbar. Besonders vorteilhaft ist die kurze Dauer der Prüfungen für Testläufe mit Reifen mit unterschiedlich angeordneten und ausgeführten Spikes, um beim gleichen Belagstyp festzustellen, welche Spikereifen welchen Einfluss auf den Belagsabrieb aufweisen.

Bei einer bevorzugten Ausführungsform der Erfindung ist der Prüfstand in einem klimatisierten Raum untergebracht, sodass ganzjährig unter konstanten Klimabedingungen (Temperatur, Luftfeuchtigkeit) geprüft werden kann. Gerade diese Maßnahme ist für die Ermittlung des Einflusses unterschiedlich angeordneten und ausgeführten Spikes auf den Belagsabrieb besonders wichtig.

Es hat sich herausgestellt, dass es ausreicht, wenn gemäß der Erfindung die Belagsplatte nur an einer Stelle der Innenfläche der Trommel eingesetzt wird. Nachdem die Trommel einen Innendurchmesser in der Größenordnung von vier Metern aufweist, wird die an einer Stelle des Umfanges befindliche Belagsplatte innerhalb weniger Minuten sehr oft überrollt und es ist innerhalb dieses Zeitraums ein aussagekräftiges Ergebnis erzielbar.

Gemäß der Erfindung erstreckt sich die Belagsplatte nur über 20 cm bis 30 cm des Umfanges der Innenfläche. Nachdem die Innenfläche der Trommel kaum breiter zu sein braucht, ist der Ein- und Ausbau der klein dimensionierten Belagsplatte problemlos und schnell durchführbar.

Die übrige Innenfläche der Trommel wird mit einem Asphalt belegt. Der Abrieb des Asphaltbelages ist nicht von Belang, bei Bedarf kann dieser jedoch nach einer Vielzahl von Prüfläufen ersetzt werden.

Beim erfindungsgemäßen Verfahren können der Schräglaufwinkel und der Sturz des Spikereifens und die Last, mit welcher der Spikereifen auf die Trommelinnenfläche aufgesetzt wird, und ferner die Antriebskraft des Spikereifens gewählt und eingestellt werden. Es ist daher möglich, Prüfläufe unter den unterschiedlichen Bedingungen, wie sie auch in der Praxis vorkommen, durchzuführen und praxisrelevante Ergebnisse zu erzielen.

Der zu prüfende Belag wird in der Form einer oder mehrerer Belagsplatten in einem Prüfkörper gehalten. Um ein komfortables Einsetzen und Entfernen der Belagsplatte bzw. der Belagsplatten sicherzustellen kann gemäß der Erfindung der Prüfkörper einen vorzugsweise rechteckigen Rahmen aufweisen, in welchem der bzw. die Belagsplatte(n) eingesetzt ist bzw. sind und welcher die Belagsplatte(n) hält. Dieser Prüfkörper wird vorzugsweise an einer Halterung, welche an der Innenfläche der Trommel befestigt ist, fixiert.

Weitere Merkmale, Vorteile und Einzelheiten der Erfindung werden nun anhand der schematischen Zeichnung, die ein Ausführungsbeispiel darstellt, näher beschrieben. Dabei zeigen
- Fig. 1: eine Schrägansicht einer in einem Prüfstand angeordneten Trommel und
- Fig. 2: eine Ansicht eines Abschnittes der Innenfläche der Trommel.

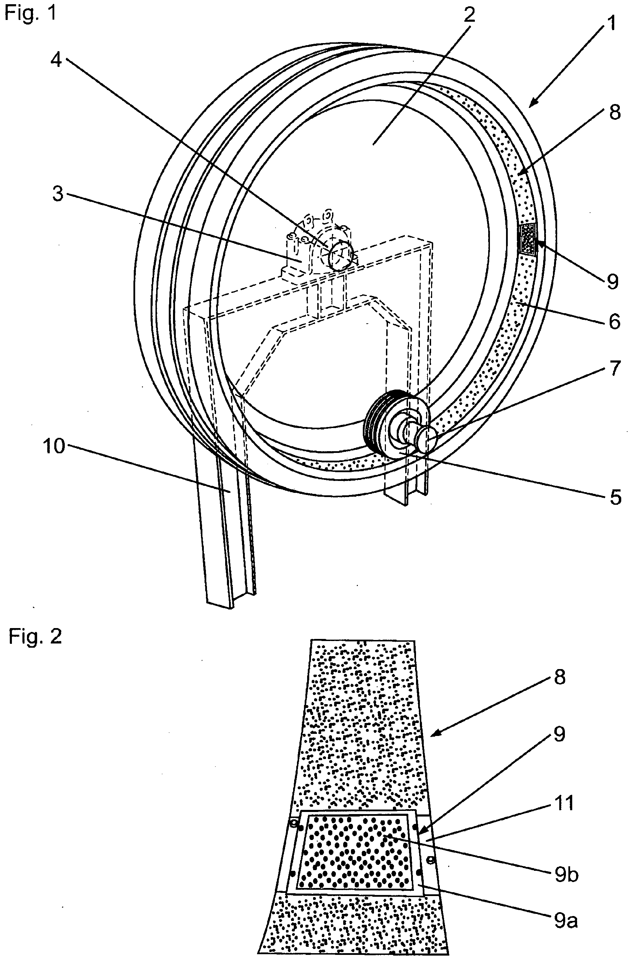

Prüfstände für Fahrzeugluftreifen, bei welchen der zu prüfende Reifen an der zylindermantelförmigen Innenfläche einer Trommel abrollt, sind an sich bekannt. Eine gemäß der Erfindung ausgeführte Trommel 1 zeigt Fig. 1. Die Trommel 1 ist ein Bestandteil eines Prüfstandes, dessen weitere Bestandteile nicht dargestellt sind und gemäß dem Stand der Technik ausgeführt sein können. Der Prüfstand verfügt über eine elektronische Steuerung, die die Einstellung und Änderung der Trommelparameter und der Reifenparameter, wie noch beschrieben wird, gestattet.

Die eine Seite der Trommel 1 ist mit einer Rückwand 2 versehen, an der anderen Seite ist die Trommel 1 frei zugänglich. An einem in Fig. 1 angedeuteten Träger 10 ist ein ebenfalls nur angedeutetes Trommellager 3 befestigt, in welchem eine an der Rückwand 2 befestigte Welle 4 drehbar gelagert ist. Die Welle 4 ist mittels eines Antriebsmotors, welcher nicht gezeigt ist, in bekannter Weise in Drehbewegung versetzbar. Die Rotationsgeschwindigkeit der Trommel 1 ist einstellbar und veränderbar. An einer weiteren nicht gezeigten Welle ist eine Felge 7 für einen Fahrzeugluftreifen 5, welcher ein Spikereifen ist, angeordnet. Diese Welle und damit der Fahrzeugluftreifen 5 sind über einen nicht dargestellten Antriebsmotor in Drehung versetzbar, wobei die Drehgeschwindigkeit und die Antriebskraft des Fahrzeugluftreifens frei wählbar und einstellbar sind. Die Lagerung der Welle erfolgt weiters derart, dass der Schräglaufwinkel und der Sturz des Fahrzeugluftreifens 5 und die Größe der Belastung des Untergrundes durch den Fahrzeugluftreifens 5 veränderbar und einstellbar sind.

An der Innenfläche 8 der Trommel 1 ist ein herkömmlicher Asphaltbelag 6, aufgebracht, welcher an zumindest einer Stelle im Umfang der Innenfläche 8, wie in Fig. 1 gezeigt, über seine Breite und eine Länge von insbesondere 20 cm bis 30 cm unterbrochen ist. An dieser Stelle wird in der durch die Unterbrechung gebildeten Vertiefung eine rechteckige rahmenartige Halterung 11 befestigt, beispielsweise mit der Trommel 1 verschraubt. In die Halterung 11 kann ein plattenförmiger Prüfkörper 9 eingesetzt und an diesem befestigt werden. Der Prüfkörper 9 besteht aus einem metallischen Rahmen 9a, in welchem eine oder mehrere Platte(n) 9b aus dem zu prüfenden Belag eingesetzt ist bzw. sind, welcher ein Gestein bzw.in einer Matrix eingebetteten Gesteinsbruch oder Kies enthält. Im Ausführungsbeispiel gemäß Fig. 2 ist eine Belagsplatte 9b dargestellt. Die Oberfläche der Belagsplatte 9b ist insbesondere strukturiert ausgeführt, beispielsweise mit regelmäßigen oder unregelmäßigen Erhebungen versehen.

Vor der Durchführung eines Testlaufes wird die trockene Belagsplatte 9b gewaschen und anschließend getrocknet und gewogen, in den Rahmen 9a eingesetzt und mit diesem an der Halterung 11 an der Innenfläche der Trommel 1 montiert. Der auf der Felge 7 montierte Spikereifen 5 wird in der Trommel 1 auf der Innenfläche 8 positioniert. Die Trommel 1 und der Spikereifen 5 werden entsprechend den vorgegebenen und eingestellten Geschwindigkeiten in Drehbewegung versetzt. Während des gesamten Prüflaufes kann eine Bewässerung der Innenfläche 8 erfolgen. Der Spikereifen 5 wird ferner entsprechend der gewählten Last auf die Innenfläche 8 gedrückt, Antriebskraft, Schräglauf und Sturz werden entsprechend den Vorgaben eingestellt. Um zu verhindern, dass der Prüfkörper immer an derselben Stelle überrollt wird kann während des Prüflaufes das Fahrzeugrad in Querrichtung verstellt werden. Nach dem Anhalten der Innentrommel 1 wird der Prüfkörper 9 gelöst, die Belagsplatte 9b wird entnommen und mit Wasser gesäubert sowie getrocknet. Anschließend wird die Belagsplatte 9b gewogen, um ihren Gewichtsverlust festzustellen.

Der Prüfstand ist in einem klimatisierten Raum untergebracht, sodass ganzjährig unter konstanten Klimabedingungen geprüft werden kann. Wechselnde Klimaeinflüsse, die das Ergebnis von Outdoorversuchen beeinflussen, sind daher bei der Prüfung mit einem erfindungsgemäßen Prüfstand ausgeschlossen.

Das Verfahren gemäß der Erfindung gestattet, eine Ermittlung des Abriebs von Straßenbelägen durch Spikereifen unter genau einstellbaren und reproduzierbaren Bedingungen und unabhängig von äußeren Witterungsverhältnissen. Die Erfindung ist daher vor allem auch dafür einsetzbar, Reifen mit unterschiedlich angeordneten und ausgeführten Spikes unter Verwendung eines bestimmten Belagstyp zu prüfen, um derart festzustellen, welche Spikes in welcher Anordnung welchen Einfluss auf den Abrieb dieses Belages nehmen.

### Bezugsziffernliste

- 1 .......................: Innentrommel
- 2.......................: Rückwand
- 3.......................: Trommellager
- 4.......................: Welle
- 5.......................: Fahrzeugluftreifen
- 6.......................: Fahrbahnbelag
- 7.......................: Felge
- 8.......................: Innenfläche
- 9.......................: Prüfkörper
- 9a.....................: Rahmen
- 9b.....................: Belagsplatte
- 10.....................: Träger
- 11.....................: Halterung

## Patentansprüche

1. Verfahren zum Feststellen des Abriebes eines Straßenbelages durch Spikereifen (5) in einem Prüflauf durch Überfahren des zu prüfenden Belages, welcher sich auf der Innenfläche (8) einer in Drehbewegung versetzten Trommel (1) befindet,
**dadurch gekennzeichnet,**
**dass** auf die Innenfläche (8) der Trommel (1) ein *herkömmlichen Asphaltbelag* aufgebracht wird, welcher an zumindest einer Stelle im Umfang der Innenfläche (8) unterbrochen wird, wobei der zu prüfende, vom Fahrbahnbelag abweichend ausgeführte Belag Gestein oder in einer Matrix eingebetteten Gesteinsbruch oder Kies enthält und als Belagsplatte oder Belagsplatten (9b) lediglich lokal an dieser Stelle bzw. diesen Stellen der Innenfläche (8) der Trommel (1) wieder entfernbar eingesetzt wird und vor und nach einem Prüflauf gewogen wird.

2. Verfahren nach Anspruch 1, **dadurch gekennzeichnet, dass** der Prüflauf in einem klimatisierten Raum durchgeführt wird.

3. Verfahren nach Anspruch 1 oder 2, **dadurch gekennzeichnet, dass** die Belagsplatte(n) (9b) nur an einer Stelle der Innenfläche (8) der Trommel (1) eingesetzt wird bzw. werden.

4. Verfahren nach einem der Ansprüche 1 bis 3, **dadurch gekennzeichnet, dass** sich die Belagsplatte(n) (9b) über 20 bis 30 cm des Umfanges der Innenfläche (8) erstreckt bzw. erstrecken.

5. Verfahren nach einem der Ansprüche 1 bis 4, **dadurch gekennzeichnet, dass** der Schräglaufwinkel und/oder der Sturz des Spikereifens (5) und/oder die Belastung der Innenfläche (8) der Trommel (1) und/oder die Antriebskraft des Spikereifen (5) wählbar und einstellbar sind.

## Claims

1. Method for determining the wear of a rood covering due to spiked tyres (5) in a test run by travelling over the covering to be tested, which covering is situated on the inner face (8) of a drum (1) set in a rotational movement,
**characterized in that**
a *conventional asphalt covering* is applied to the inner face (8) of the drum and is interpreted at at least one point in the circumference of the inner face (8), wherein the covering to be tested and which is made to differ from the roadway covering contains stones or broken stones, which are embedded in a matrix, or gravel and is used as a covering panel or covering panels (9b) only locally at this point or these points of the inner face (8) of the drum (1) in such a way as to be removable again and is weighed before and after a test run.

2. Method according to Claim 1, **characterized in that** the test run is carried out in a climate-controlled space.

3. Method according to Claim 1 or 2, **characterized in that** the covering panel(s) (9b) is or are used only at one point of the inner face (8) of the drum (1).

4. Method according to one of Claims 1 to 3, **characterized in that** the covering panel(s) (9b) extends or extend over 20 to 30 cm of the circumference of the inner face (8).

5. Method according to one of Claims 1 to 4, **characterized in that** the slip angle and/or the cumber of the spiked tyre (5) and/or the loading of the inner face (8) of the drum (1) and/or the driving force of the spiked tyre (5) are/is selectable and adjustable.

## Revendications

1. Procédé de détermination de l'abrasion d'un revêtement routier par des bandages (5) de roue à clous dans un déplacement de test, par passage sur le revêtement à tester situé sur la surface intérieure (8) d'un tambour (1) mis en rotation,
**caractérisé en ce que**
un revêtement d'asphalte habituel est appliqué sur la surface intérieure (8) du tambour (1) et est interrompu en au moins un emplacement de la périphérie de la surface intérieure (8),
**en ce que** le revêtement à tester, différent du revêtement de chaussée, contient des pierres, des concassés ou graviers incorporés dans une matrice, est utilisé comme plaque de revêtement ou plaques de revêtements (9b) remplaçables, uniquement localement, en cet emplacement ou ces emplacements de la surface intérieure (8) du tambour (1) et est pesé avant et après un passage de test.

2. Procédé selon la revendication 1, **caractérisé en ce que** le passage de test est exécuté dans un espace climatisé.

3. Procédé selon les revendications 1 ou 2, **caractérisé en ce que** la ou les plaques de revêtement (9b) ne sont utilisées qu'en un seul emplacement de la surface intérieure (8) du tambour (1).

4. Procédé selon l'une des revendications 1 à 3, **caractérisé en ce que** la ou les plaques de revêtement (9b) s'étendent sur 20 à 30 cm de la périphérie de la surface intérieure (8).

5. Procédé selon l'une des revendications 1 à 4, **caractérisé en ce que** l'angle d'obliquité et/ou l'attaque du bandage (5) de roue à clous et/ou la sollicitation de la surface intérieure (8) du tambour (1) et/ou la force d'entraînement du bandage (5) de roue à clous peuvent être sélectionnés et réglés.
